(19) Europäisches Patentamt
European Patent Office
Office européen des brevets

(11) Veröffentlichungsnummer: **0 326 887 B1**

(12) **EUROPÄISCHE PATENTSCHRIFT**

(45) Veröffentlichungstag der Patentschrift: **16.09.92**

(51) Int. Cl.$^{5}$: **C07D 471/04**, A61K 31/435, //(C07D471/04,221:00,205:00)

(21) Anmeldenummer: **89101079.5**

(22) Anmeldetag: **23.01.89**

Die Akte enthält technische Angaben, die nach dem Eingang der Anmeldung eingereicht wurden und die nicht in dieser Patentschrift enthalten sind.

# (54) Beta-Lactam-Antibiotika, Verfahren zu ihrer Herstellung und ihre Verwendung als und in Arzneimitteln.

(30) Priorität: **03.02.88 DE 3803169**

(43) Veröffentlichungstag der Anmeldung:
**09.08.89 Patentblatt 89/32**

(45) Bekanntmachung des Hinweises auf die Patenterteilung:
**16.09.92 Patentblatt 92/38**

(84) Benannte Vertragsstaaten:
**AT BE CH DE ES FR GB GR IT LI NL SE**

(56) Entgegenhaltungen:
**EP-A- 0 164 113**
**EP-A- 0 195 947**
**EP-A- 0 197 294**
**EP-A- 0 225 634**
**WO-A-85/04878**

(73) Patentinhaber: **BAYER AG**

**W-5090 Leverkusen 1 Bayerwerk(DE)**

(72) Erfinder: **Schmidt, Gunter, Dr.**
**Pahlkestrasse 63**
**W-5600 Wuppertal 1(DE)**

Erfinder: **Hartwig, Wolfgang, Dr.**
**Pahlkestrasse 3**
**W-5600 Wuppertal 1(DE)**
Erfinder: **Schröck, Wilfried,Dr.**
**Pahlkestrasse 33**
**W-5600 Wuppertal 1(DE)**
Erfinder: **Endermann, Rainer, Dr.**
**In den Birken 152a**
**W-5600 Wuppertal 1(DE)**
Erfinder: **Metzger, Karl Georg, Dr.**
**Pahlkestrasse 75**
**W-5600 Wuppertal 1(DE)**
Erfinder: **Haller, Ingo, Dr.**
**Dornröschenweg 4**
**W-5600 Wuppertal 1(DE)**
Erfinder: **Zeiler, Hans-Joachim, Dr.**
**Am Rohm 86**
**W-5600 Wuppertal 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft $\beta$-Lactam-Antibiotika, Verfahren zu ihrer Herstellung sowie ihre Verwendung als Arzneimittel und in Arzneimitteln, insbesondere als antibakteriell, oral wirksame Antibiotika.

Es ist bekannt, daß verschiedene Vertreter von 7-$\alpha$-Aminoacylcephalosporinen mit unterschiedlichen Substituenten in der 3-Stellung des Moleküls antibiotisch wirken, so z.B. Cephalexin [7-(D-$\alpha$-Phenylglycylamido)-3-methyl-3-cephem-4-carbonsäure], Cefaclor [7-(D-$\alpha$-Pheylglycylamido)-3-chlor-3-cephem-4-carbonsäure] (vgl. GB-Patent 1 174 335; DE-OS 24 08 698 und DE-OS 27 28 578).

Weiterhin sind $C_3$-substituierte Cephalosporine als oral wirksame Verbindungen in DE-OS 35 08 258 und DE-OS 35 09 618 beschrieben.

Die vorliegende Erfindung betrifft heteroanellierte Phenylglycin-$\beta$-Lactam-Antibiotika der allgemeinen Formel (I)

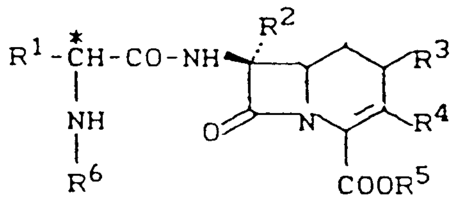

(I)

in welcher

R[1] - für eine Gruppe der Formel

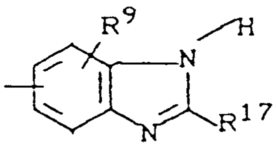 , 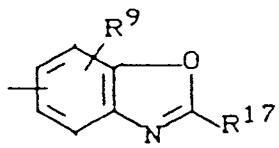 , 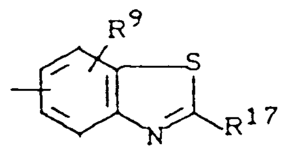

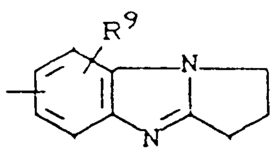 , 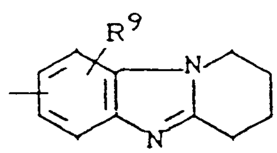 , 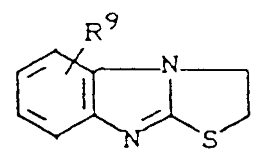

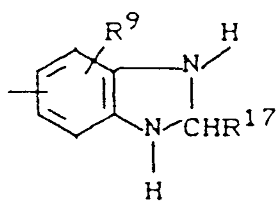 , 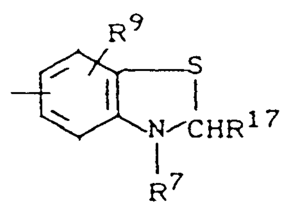 , 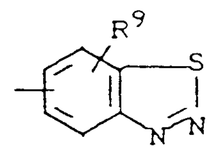 ,

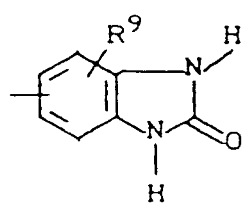 , 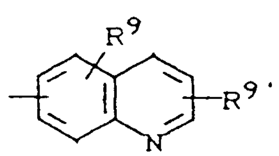 , 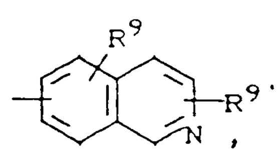 ,

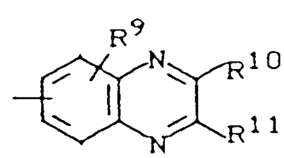 , 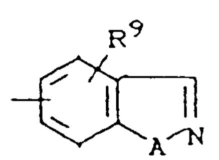 , 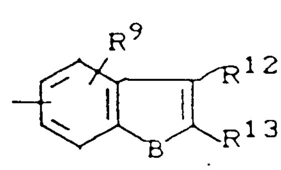

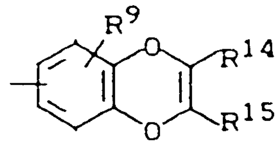

steht,
worin

$R^7$ - für Wasserstoff steht, oder
- für geradkettiges, verzweigtes oder cyclisches, gegebenenfalls durch Fluor, Amino, Hydroxy oder Phenyl substituiertes Alkyl oder Alkenyl mit bis zu 6 Kohlenstoffatomen steht,

$R^9$, $R^{9'}$ gleich oder verschieden sind und
- für Wasserstoff stehen, oder
- für geradkettiges oder verzweigtes Alkyl, Alkoxy oder Alkylthio mit jeweils bis zu 4 Kohlenstoffatomen stehen, oder
- für Trifluormethyl oder Trifluormethoxy stehen, oder
- für Hydroxy, Nitro, Cyano, Fluor oder Chlor stehen, oder
- für Amino, Methylamino, Dimethylamino stehen,

$R^{10}$, $R^{11}$ gleich oder verschieden sind und für Wasserstoff stehen, oder
- für Phenyl stehen, das gegebenenfalls substituiert ist durch Chlor, Fluor, Alkyl mit bis zu 4 Kohlenstoffatomen, Methoxy, Methylthio, Trifluormethyl, Trifluormethoxy, Trifluormethylthio, Nitro, Cyano, Amino oder Dimethylamino, oder
- für Amino, Methylamino, Dimethylamino stehen, oder
- für Hydroxy stehen, oder
- für Alkoxy mit bis zu 4 Kohlenstoffatomen stehen, oder
- für Benzoyloxy oder Acetyloxy stehen, oder
- für geradkettiges, verzweigtes oder cyclisches Alkyl oder Alkenyl mit jeweils bis zu 6 Kohlenstoffatomen stehen, das substituiert sein kann, durch Fluor, Chlor, Methoxy, Methylthio, Trifluormethoxy oder Cyano,

$R^{12}$, $R^{13}$ gleich oder verschieden sind und
- für Wasserstoff stehen, oder
- für Phenyl stehen, das gegebenenfalls substituiert ist durch Fluor, Chlor, Alkyl mit bis zu 4 Kohlenstoffatomen, Methoxy, Methylthio, Trifluormethyl, Trifluormethoxy, Trifluormethylthio, Nitro, Cyano, Amino oder Dimethylamino, oder
- für Pyridyl, Thienyl, Furyl, Pyrimidyl oder Hydroxy stehen, oder
- für Amino, Methylamino, Dimethylamino stehen, oder
- für Alkoxy mit bis zu 4 Kohlenstoffatomen stehen, oder
- für Benzoyloxy oder Acetyloxy stehen, oder
- für Benzoyl oder Acetyl stehen, oder
- für Alkoxycarbonyl mit bis zu 4 Kohlenstoffatomen stehen, oder
- für geradkettiges, verzweigtes oder cyclisches Alkyl oder Alkenyl mit bis zu 6 Kohlenstoffatomen stehen, das gegebenenfalls substituiert ist durch Fluor, Chlor, Methoxy, Methylthio, Trifluormethoxy, Cyano, Phenyloxy oder Benzyloxy,

$R^{14}$, $R^{15}$ gleich oder verschieden sind und
- für Wasserstoff stehen, oder
- für geradkettiges, verzweigtes oder cyclisches Alkyl oder Alkenyl mit bis zu 6 Kohlenstoffatomen stehen, das gegebenenfalls substituiert ist durch Fluor, Chlor, Methoxy, Methylthio, Trifluormethoxy oder Cyano, oder
- für Phenyl stehen, das gegebenenfalls substituiert ist durch Fluor, Chlor, Alkyl mit bis zu 4 Kohlenstoffatomen, Methoxy, Methylthio, Trifluormethyl, Trifluormethoxy, Trifluormethylthio, Nitro, Cyano, Amino oder Dimethylamino, oder
- für Alkoxycarbonyl mit bis zu 4 Kohlenstoffatomen stehen,

A - für O, S oder $-NR^{19}$ steht,

B - für O oder $-NR^{16}$ steht,

$R^{16}$ für Wasserstoff steht, oder
- für Phenyl steht, oder
- für geradkettiges oder verzweigtes Alkyl mit bis zu 4 Kohlenstoffatomen steht,

$R^{17}$ für Wasserstoff steht, oder
- für geradkettiges oder verzweigtes Alkyl oder Alkenyl mit jeweils bis zu 4 Kohlenstoffatomen steht, das gegebenenfalls substituiert ist durch Fluor, Chlor, Alkoxy mit bis zu 4 Kohlenstoffatomen, Hydroxy, Carboxy, Phenyl, Sulfo, Amino, Alkylamino, Dialkylamino mit jeweils bis zu 3 Kohlenstoffatomen je Alkylgruppe, Phenylamino, Benzylamino oder durch Acetylamino, oder
- für Fluor, Chlor oder Brom steht, oder
- für Alkoxy oder Alkylthio mit jeweils bis zu 4 Kohlenstoffatomen steht, oder
- für Phenyl steht, oder
- für Amino, Alkylamino, Dialkylamino mit jeweils bis zu 3 Kohlenstoffatomen je Alkylgruppe steht, oder

$R^{18}$ - die gleiche Bedeutung hat wie $R^{17}$ und mit diesem gleich oder verschieden sein kann und

$R^{19}$ - die gleiche Bedeutung hat wie $R^{16}$ und mit diesem gleich oder verschieden ist,

$R^2$ - für Wasserstoff steht, oder
- für Methoxy steht, oder
- für Formamido und Hydroxylamino steht,

$R^3$ - für Wasserstoff, Hydroxy, Methyl oder Methoxy steht,

$R^4$ - für Wasserstoff, Chlor, Methyl, Methoxy, Methoxymethyl, Vinyl, cis-Propenyl oder Acetyloxymethyl steht, oder

$R^5$ - für Wasserstoff steht, oder
- für einen Rest der Formel $-CH_2-OCOCH_3$,

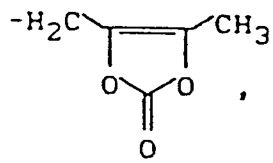 ,

$-CH(CH_3)-OCOOC_2H_5$ oder $-CH_2-OCO-C(CH_3)_3$ steht,
und

$R^6$ - für Wasserstoff steht,

und deren pharmazeutisch verträglichen Natriumsalze.

Die Verbindungen der Formel I können als freie Säuren, Ester, als innere Salze oder als nicht-toxische pharmazeutisch verträgliche Salze der sauren Carboxylgruppen, wie Natrium-, Kalium-, Magnesium-, Calcium-, Aluminium- oder Ammoniumsalze, mit Aminen wie Di-, Tri-niedrigalkylaminen, Procain, Dibenzylamin, N,N'-Dibenzylethylendiamin, N-Benzyl-$\beta$-phenyl-ethylamin, N-Methyl und N-Ethylmorpholin, 1-Ephenamin, Dihydroabietylamin, N,N'-Bis-dehydroabietylethylendiamin, N-Niedrigalkylpiperidin und anderen Aminen, die zur Bildung von Salzen von Penicillinen und Cephalosporinen verwendet werden können, vorliegen.

Wegen der Anwesenheit des mit * bezeichneten asymmetrischen Kohlenstoffatoms schließen die neuen $\beta$-Lactamantibiotika der Formel (I) die D-, L- und D,L-Form ein. Bevorzugt sind die D-Formen der erfindungsgemäßen Verbindungen der allgemeinen Formel (I).

Sowohl die Diastereomerengemische als auch die D-Form und L-Form der erfindungsgemäßen Verbindungen können zur Behandlung bakterieller Infektionskrankheiten eingesetzt werden.

Darüberhinaus wurde ein Verfahren zur Herstellung der erfindungsgemäßen heteroanellierten Phenylglycin-$\beta$-Lactam-Antibiotika der allgemeinen Formel (I) gefunden, das dadurch gekennzeichnet ist, daß man

Carbonsäuren der allgemeinen Formel (II)

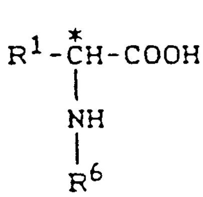

(II)

in welcher
$R^1$ und $R^6$ die oben angegebene Bedeutung haben,
nach Aktivierung der Carboxylgruppe durch Überführung in ein gemischtes Anhydrid, beispielsweise mit Chlorameisensäureethylester oder Chlorameisensäureisobutylester oder Methansulfonsäurechlorid, oder durch Überführen in das Säurehalogenid, oder durch Überführen in einen aktivierten Ester, beispielsweise mit Dicyclohexylcarbodiimid (DCC), gegebenenfalls in Anwesenheit von N-Hydroxybenztriazol,
mit den $\beta$-Lactamaminen der allgemeinen Formel (III),

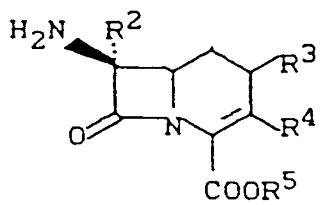

(III)

in welcher
$R^2$, $R^3$, $R^4$ und $R^5$ die oben angegebene Bedeutung haben,
umsetzt,
dann gegebenenfalls Schutzgruppen abspaltet und die gewünschten Salze oder aus den Salzen die freien Säuren herstellt.

Das erfindungsgemäße Verfahren kann durch das folgende Formelschema erläutert werden:

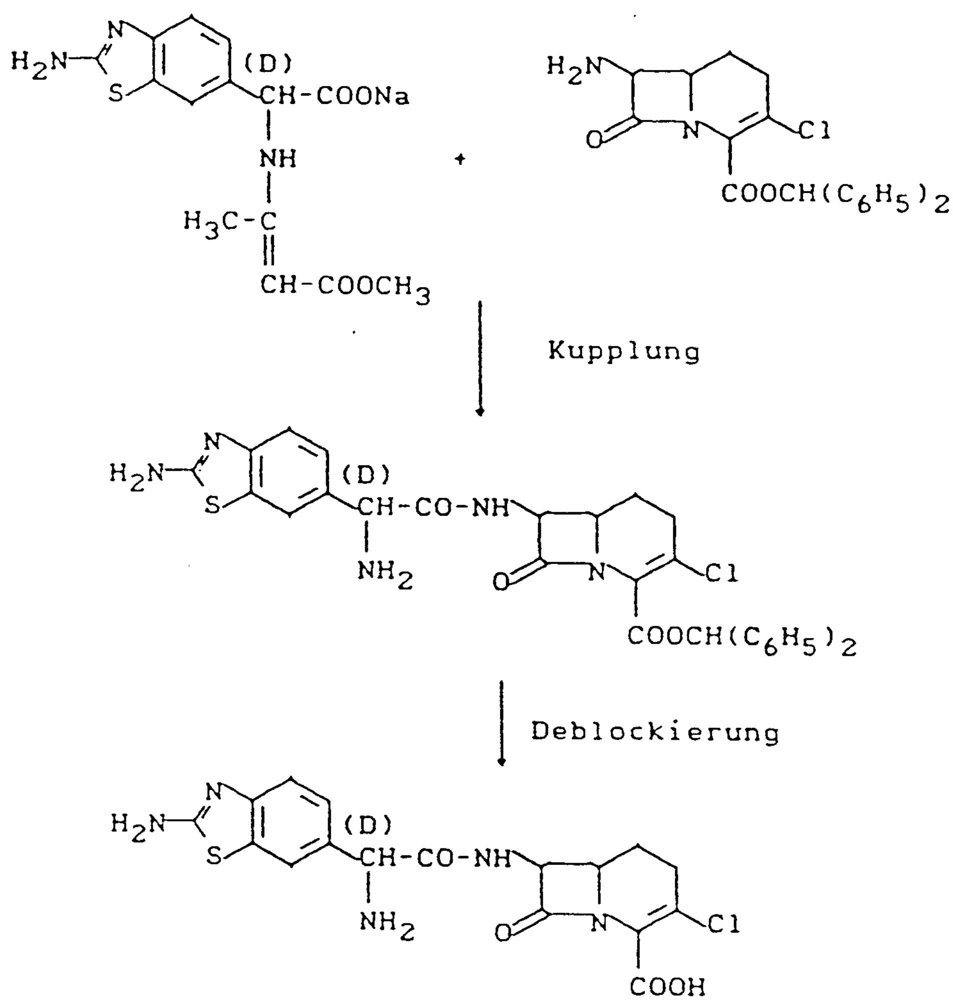


Bei der Durchführung des Verfahrens hat es sich als vorteilhaft erwiesen, die Carbonsäure zu aktivieren

und dann mit den β-Lactamaminen, die als Salze mit Amin in Lösung gebracht werden, zu kuppeln. Besonders vorteilhaft ist die Aktivierung mit Sulfonsäurederivaten der allgemeinen Formel (IV) oder mit Chlorameisensäureestern, bevorzugt Chlorameisensäureethylester, zu Anhydriden der allgemeinen Formel (Va, b), wie im folgenden Reaktionsschema verdeutlicht wird.

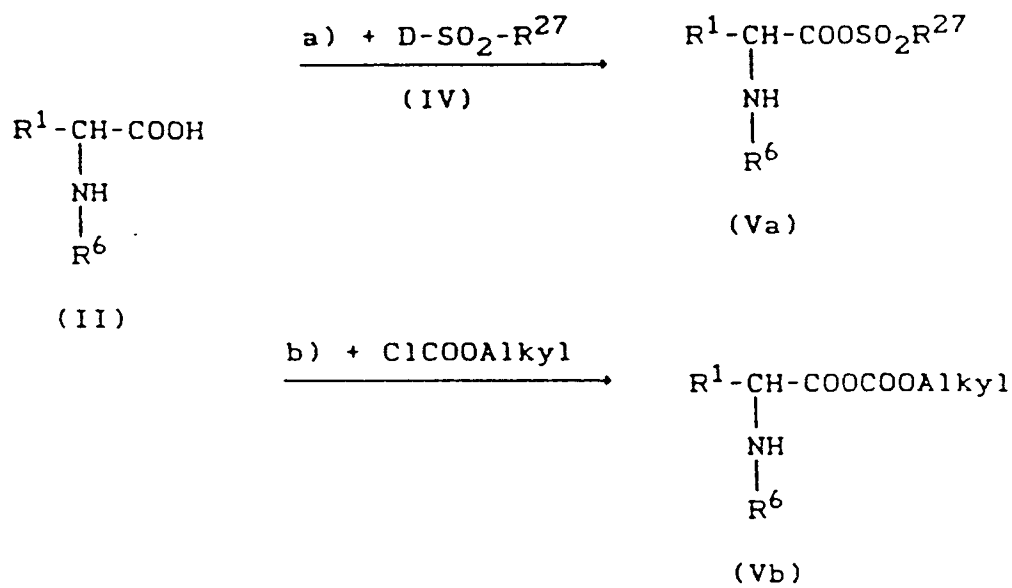


Hierbei steht in der Formel (IV) bzw. (Va)

D - für den Rest $R^{27}$-$SO_2$-O- oder Halogen und

$R^{27}$ - für Alkyl mit bis zu 10 Kohlenstoffatomen, das gegebenenfalls substituiert ist durch Fluor, Chlor, Cyano, Phenyl, Alkoxycarbonyl oder Alkoxy mit jeweils bis zu 4 Kohlenstoffatomen, oder
- für Phenyl, das gegebenenfalls substituiert ist durch Fluor, Chlor, Brom, Cyano, Alkyl, Alkoxy, Alkylthio, Alkoxycarbonyl oder Alkylcarbonyl mit jeweils bis zu 4 Kohlenstoffatomen, Nitro, Trifluormethyl oder Phenyl

Wenn $R^{27}$ substituiert ist, sind bevorzugt ein bis drei Substituenten, besonders bevorzugt die oben genannten vorhanden.

Ganz besonders bevorzugt stellt $R^{27}$ einen Methyl- oder p-Tolylrest dar.

Die gemischten Anhydride der allgemeinen Formel (Va, b) werden hergestellt, indem man die Carbonsäuren der allgemeinen Formel (II) und 1- bis 1,4-Äquivalente eines Amins in einem Lösemittel löst und mit 1- bis 1,2-Äquivalenten eines Sulfonsäurederivates der Formel (IV) oder eines Chlorameisensäureesters reagieren läßt.

Als Lösemittel eignen sich alle Solventien, die sich unter den Reaktionsbedingungen nicht verändern. Hierzu gehören bevorzugt Ether wie beispielsweise Diethylether, Dioxan oder Tetrahydrofuran, oder Chlorkohlenwasserstoffe wie Methylenchlorid, Chloroform oder Tetrachlorkohlenstoff, oder Amide wie Dimethylformamid oder Hexamethylphosphorsäurtriamid, oder Acetonitril, oder Aceton. Ebenso ist es möglich, Gemische der genannten Lösemittel einzusetzen, gegebenenfalls in Mischung mit Wasser.

Als Amine eignen sich tertiäre Amine wie beispielsweise Triethylamin, Ethyldiisopropylamin oder Tributylamin, aber auch sterisch gehinderte sekundäre Amine wie beispielsweise Diisopropylamin. Ebenso können Gemische der genannten Amine eingesetzt werden.

Die Umsetzung kann bei Temperaturen zwischen -80 °C und Raumtemperatur durchgeführt werden. Vorteilhaft wird die Aktivierung mit Methansulfonsäurechlorid in Dimethylformamid bei -40 °C bis -60 °C durchgeführt.

Zum Lösen der β-Lactamamine der allgemeinen Formel (III) können die bei der Herstellung der Verbindung der Formel (V) genannten Lösemittel oder Wasser, sowie als Base die dort genannten Amine verwendet werden.

Besonders vorteilhaft ist auch eine Aktivierung der Carbonsäure der allgemeinen Formel (II) durch Überführen in einen aktivierten Ester mit beispielsweise Dicyclohexylcarbodiimid, gegebenenfalls in Anwesenheit von N-Hydroxysuccinimid oder 1-Hydroxybenztriazol.

Als Lösemittel eignen sich hierbei alle Solventien, die sich auch für die Herstellung von Anhydriden der allgemeinen Formel (V) eignen und dort bereits aufgeführt sind. Die Umsetzungen können bei Temperaturen zwischen -30°C und +100°C durchgeführt werden. Vorteilhaft wird mit 1-Hydroxybenztriazol und Dicyclohexylcarbodiimid in Dimethylformamid bei Raumtemperatur 2 bis 6 Stunden aktiviert. Dann vom ausgefallenen Dicyclohexylharnstoff abgesaugt und mit dem β-Lactamamin der allgemeinen Formel (III) in Form einer Lösung ihres Aminsalzes, innerhalb von 2 bis 24 Stunden umgesetzt. Zum Lösen der β-Lactamamine der allgemeinen Formel (III) können die bei der Herstellung der Verbindung der Formel (V) genannten Lösemittel sowie als Base die dort genannten Amine verwendet werden.

Die als Ausgangsverbindungen eingesetzten Carbonsäuren der allgemeinen Formel (II) sind bekannt oder können nach bekannten Methoden hergestellt werden [DE-OS 35 09 618; DE-OS 35 08 258].

Die als Ausgangsstoffe eingesetzten β-Lactamamine der allgemeinen Formel (III) sind bekannt oder können nach bekannten Methoden hergestellt werden [EP-PS 0 154 253; EP-PS 0 025 602; EP-PS 0 027 882; EP-PS 0 075 805; EP-PS 0 014 475; EP-PS 0 014 476; Chem. Pharma. Bull. 28, (5), 1563 (1980)].

Die stereochemisch einheitliche D- bzw. L-Formen der erfindungsgemäßen Verbindungen der Formel (I) erhält man, wenn man die Diastereomerengemische durch HPLC-Chromatographie trennt.

Andererseits erhält man die reine D- bzw. L-Form (bevorzugt die D-Form), wenn man bereits auf der Stufe der racemischen Aminosäure der Formel (II) eine chemische Racematspaltung, z.B. mit Dehydroabietylamin, Phenylethylamin oder Camphersulfonsäure, oder eine Racematspaltung z.B. über N-Acetyl-aminosäurederivate, z.B. mit Subtilisin, Penicillinacylase oder Schweinenierenacylase, vornimmt und anschließend die stereochemisch einheitlichen D- bzw. L-Formen der Verbindungen der Formel (II) in angegebener Weise umsetzt.

Als neue erfindungsgemäße Wirkstoffe aus der Reihe der Cephalosporine seien im einzelnen, neben den experimentellen Beispielen folgende Verbindungen genannt:

Tabelle 1

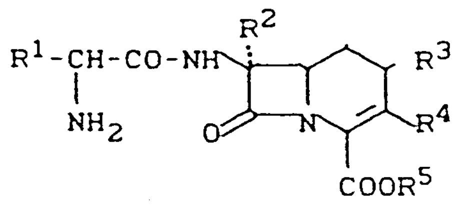

$R^2$ und $R^5$ = H

| $R^1$ | $R^3$ | $R^4$ |
|---|---|---|
| 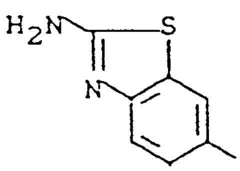 | H | Cl |
| 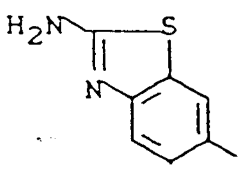 | H | $CH_3$ |
| 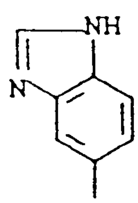 | H | Cl |

Fortsetzung Tabelle

| $R^1$ | $R^3$ | $R^4$ |
|---|---|---|
| 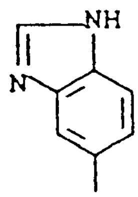 | OH (β) | H |
| 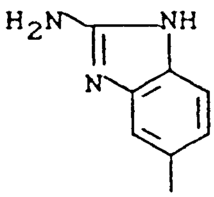 | OH (β) | H |
| 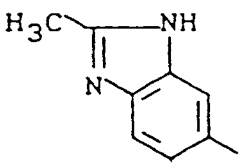 | H | Cl |
| 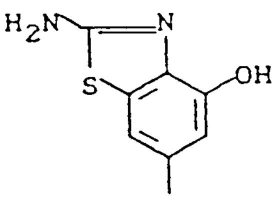 | H | Cl |

Fortsetzung Tabelle

| $R^1$ | $R^3$ | $R^4$ |
|---|---|---|
| 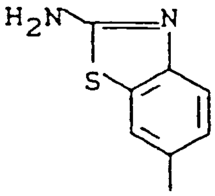 | OH (β) | H |
| 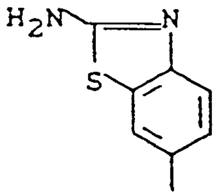 | H | $-CH=CH-CH_3$ (cis) |
| 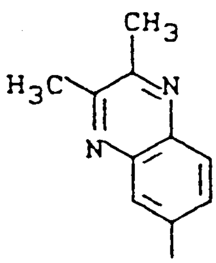 | H | Cl |

Fortsetzung Tabelle

| $R^1$ | $R^3$ | $R^4$ |
|---|---|---|
| 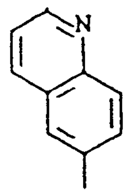 | H | Cl |
| 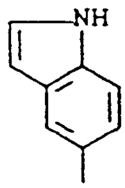 | H | Cl |
| 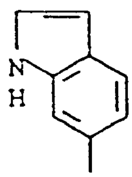 | H | Cl |
| 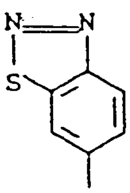 | OH (β) | H |
| 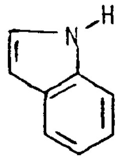 | H | $-CH=CH-CH_3$ (cis) |

Fortsetzung Tabelle

| $R^1$ | $R^3$ | $R^4$ |
|---|---|---|
| 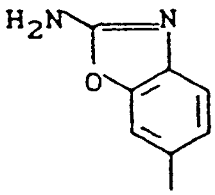 | H | Cl |
| 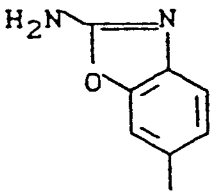 | $-CH_3$ (β) | H |
| 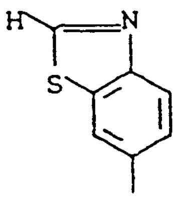 | H | Cl |
| 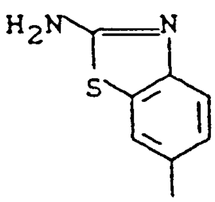 | $-CH_3$ (α) | H |

Als Wirkstoffe seien im einzelnen folgende Carbacephem-Derivate genannt:

(6R, 7S)-(D)-7-[(2-Aminobenzothiazol-5-yl)glycylamido]-3-chlor-1-azabicyclo[4,2,0]oct-2-en-8-on-2-carbonsäure

(6R, 7S)-(D)-7-[(Benzimidazol-5(6)-yl)glycylamido]-3-chlor-1-azabicylco[4,2,0]oct-2-en-8-on-2-carbonsäure

(6R, 7S)-(D)-7-[(Benzimidazol-5(6)-yl)glycylamido]-4β-hydroxy-1-azabicyclo[4,2,0]oct-2-en-8-on-2-carbonsäure

(6R, 7S)-(D)-7-[(2-Amino-1H-benzimidazol-5(6)-yl)glycylamido]-4β-hydroxy-1-azabicyclo[4,2,0]oct-2-en-8-on-2-carbonsäure

(6R, 7S)-(D)-7-[(2-Methyl-1H-benzimidazol-5-yl)glycylamido]-3-chlor-1-azabicyclo[4,2,0]oct-2-en-8-on-2-carbonsäure

(6R, 7S)-(D)-7-[(2-Cyclopropyl-benzothiazol-6-yl)glyclyamino]-3-chlor-1-azabicyclo[4,2,0]oct-2-en-β-on-2-carbonsäure

(6R, 7S)-(D)-7-[(2-Amino-4-hydroxy-benzothiazol-6-yl)glycylamido]-3-chlor-1-azabicyclo[4,2,0]oct-2-en-8-on-2-carbonsäure

(6R, 7S)-(D)-7-[(2-Mesylamino-benzothiazol-6-yl)glycylamido]-3-[(Z)-1-propen-1-yl]-1-azabicyclo-[4,2,0]-oct-2-en-8-on-2-carbonsäure

(6R, 7S)-(D)-7-[(2-Aminobenzothiazol-6-yl)glycylamido]-4β-hydroxy-1-azabicyclo[4,2,0]oct-2-en-8-on-2-carbonsäure

(6R, 7S)-(D)-7-[(2-Aminobenzothiazol-6-yl)glycylamido]-3-[(Z)-1-propen-1-yl]-1-azabicyclo[4,2,0]oct-2-en-8-on-2-carbonsäure

(6R, 7S)-(D)-7-[(2,3-Dimethylchinoxalin-6-yl) glycylamido]-3-chlor-1-azabicyclo[4,2,0]oct-2-en-8-on-2-carbonsäure
(6R, 7S)-(D)7-[(Chinol-6-yl)glycylamido]-3-chlor-1-azabicyclo[4,2,0]oct-2-en-8-on-2-carbonsäure
(6R, 7S)-(D)-7-[(Indol-5-yl)glycylamido)-3-chlor-1-azabicyclo[4,2,0]oct-2-on-8-on-2-carbonsäure
(6R, 7S)-(D)-7-[(Indol-6-yl)glycylamido]-3-chlor-1-azabicyclo[4,2,0]oct-2-en-8-on-2-carbonsäure
(6R, 7S)-(D)-7-[1,2,3-Benzothiadiazol-6-yl)glycylamido]-4-β-hydroxy-1-azabicyclo[4,2,0]oct-2-en-8-on-2-carbonsäure
(6R, 7S)-(D)-7-[(Indol-5-yl)glycylamido]-3-[(Z)-1-propen-1-yl]-1-azabicyclo-[4,2,0]oct-2-en-8-on-2-carbonsäure
(6R, 7S)-(D)-7-[(2-Aminobenzoxazol-6-yl)glycylamido]-3-chlor-1-azabicyclo[4,2,0]oct-2-en-8-on-2-carbonsäure
(6R, 7S)-(D)-7[(2-Aminobenzoxazol-6-yl)glycylamido]-4β-methyl-1-azabicyclo[4,2,0]oct-2-en-8-on-2-carbonsäure
(6H, 7S)-(D)-7-[(Benzothiazol-6-yl)glycylamido]-3-chlor-1-azabicyclo[4,2,0]oct-2-en-8-on-2-carbonsäure
(6R, 7S)-(D)-7-[(2-Aminobenzothiazol-6-yl)glycylamido]-4α-methyl-1-azabicyclo[4,2,0]oct-2-en-8-on-2-carbonsäure

Die erfindunsgemäßen Verbindungen der allgemeinen Formel I weisen bei geringer Toxizität ein breites antibakterielles Spektrum gegen gram-positive und gram-negative Keime auf. Diese Eigenschaften ermöglichen ihre Verwendung als chemotherapeutische Wirkstoffe in der Human- und Tiermedizin.

Die erfindungsgemäßen Verbindungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder geheilt werden.

Besonders wirksam sind die erfindungsgemäßen Verbindungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:
Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Hämophilus influenzae, Citrobacter (Citrob. freundii, Citrob. divernis), Salmonella und Shigella; ferner Klebsiellen (Klebs. pneumoniae, Klebs. oxyloca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginoso, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, z.B. Mycobacterium tuberculosis. Insbesondere wirken die erfindungsgemäßen Stoffe gegen Staphylokokken, Streptokokken,Enterokokken und Haemophilus influenzae. Bei parenteraler oder insbesondere oraler Verabreichung sind die neuen Verbindungen gegen Mikroorganismen wie Staphylokokken, Streptokokken, Enterobakteriaceen, Escherichia coli, Klebsiella, Salmonella, Shigella und Proteus sehr gut wirksam.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:
Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, postoperative Windinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Menigitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:
Schwein: Coli-diarrhoe, Enterotoxämie, Sepsis, Dysenterie, Salmonellose, Metritis-Mastitis-Agalaktiae-Syndrom, Mastitis;
Wiederkäuer (Rind, Schaf, Ziege): Diarrhoe, Sepsis, Bronchopneumonie, Salmonellose, Pasteurellose, Mykoplasmose, Genitalinfektionen;
Pferd: Bronchopneumonie, Fohlenlähme, puerperale und postpuerperale Infektionen, Salmonellose;
Hund und Katze: Bronchopneumonie, Diarrhoe, Dermatitis, Otitis, Harnwegsinfekte, Prostatitis;
Geflügel (Huhn, Pute, Wachtel, Taube, Ziervögel und andere): Mycoplasmose, E. coli-Infektionen, chronische Luftwegserkrankungen, Salmonellose, Pateurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borellia, Treponema, Nocardia, Rikettsien, Yersinia, erweitert.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Zur vorliegenden Erfindung gehören auch pharmazeutische Zubereitungen in Dosierungseinheiten. Dies bedeutet, daß die Zubereitung in Form einzelner Teile, z.B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z.B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht-toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z.B. Stärken, Milchzucker, Rohrzucker, Glukose, Mannit und Kieselsäure, (b) Bindemittel, z.B. Carboxymethylcellulose, Alginate, Gelatine, Polyvinylpyrrolidon, (c) Feuchthaltemittel, z.B. Glycerin, (d) Sprengmittel, z.B. Agar-Agar, Calciumcarbonat und Natriumcarbonat, (e) Lösungsverzögerer, z.B. Paraffin und (f) Resorptionsbeschleuniger, z.B. quarternäre Ammoniumverbindungen, (g) Netzmittel, z.B. Cetyl-alkohol, Glycerinmonostearat, (h) Adsorptionsmittel, z.B. Kaolin und Bentonit und (i) Gleitmittel z.B. Talkum, Calcium- und Magnesiumstearat und feste Polyethylenglykole oder Gemische der unter (a) bis (i) aufgeführten Stoffe.

Die Tabletten, Dragees, Kapseln, Pillen und Granulate können mit den üblichen, gegebenenfalls Opakisierungsmitteln enthaltenden, Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z.B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z.B. Polyethylenglykole, Fette, z.B. Kakaofett und höhere Ester (z.B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z.B. tierische und pflanzliche Fette, Wachse, Paraffine, Stärke, Tragant, Cellulosederivate, Polyethylenglykole, Silikone, Bentonite, Kieselsäure, Talkum und Zinkoxid oder Gemische dieser Stoffe.

Puder und Sprays können neben dem oder den Wirkstoffen die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe wie Lösungsmittel, Lösungsvermittler und Emulgatoren, z.B. Wasser, Ethylalkohol, Isopropylalkohol, Ethylcarbonat, Ethylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydofurfurylalkohol, Polyethylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z.B. Wasser, Ethylalkohol, Propylenglykol, Suspendiermittel, z.B. ethoxylierte Isostearylalkohole, Polyoxyethylensorbit- und Sorbitan-Ester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmacksverbesserte Zusätze, z.B. Pfefferminzöl und Eukalyptusöl und Süßmittel, z.B. Saccharin, enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Die genannten Zubereitungen können bei Mensch und Tier entweder oral, rektal, parenteral (intravenös, intramuskulär, subkutan), intracisternal, intravaginal, intraperitoneal, lokal (Puder, Salbe, Tropfen) und zur Therapie von Infektionen in Hohlräumen, Körperhöhlen angewendet werden. Als geeignete Zubereitungen kommen Injektionlösungen, Lösungen und Suspensionen für die orale Therapie, Gele, Aufgußformulierungen, Emulsionen, Salben oder Tropfen in Frage. Zur lokalen Therapie können ophtalmologische und dermatologische Formulierungen, Silber- und andere Salze, Ohrentropfen, Augensalben, Puder oder Lösungen verwendet werden. Bei Tieren kann die Aufnahme auch über das Futter oder Trinkwasser in geeigneten Formulierungen erfolgen.

Ferner können Gele, Pulver, Puder, Tabletten, Retard-Tabletten, Premixe, Konzentrate, Granulate, Pellets, Tabletten, Boli, Kapseln, Aerosole, Sprays, Inhalate bei Mensch und Tier angewendet werden. Ferner können die erfindungsgemäßen Verbindungen in andere Trägermaterialien wie zum Beispiel Kunststoffe, (Kunststoffketten zur lokalen Therapie), Kollagen oder Knochenzement eingearbeitet werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise S bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

So kann es in einigen Fällen ausreichend sein, mit weniger als der obengenannten Menge Wirkstoff auszukommen, während in anderen Fällen die oben angeführte Wirkstoffmenge überschritten werden muß Die Festlegung der jeweils erforderlichen optimalen Dosierung und Applikationsart der Wirkstoffe kann durch jeden Fachmann aufgrund seines Fachwissens leicht erfolgen.

Die neuen Verbindungen können in den üblichen Konzentrationen und Zubereitungen zusammen mit dem Futter bzw. Lactamaseinhibitoren z.B. mit Penicillinen, die besonders penicillinasefest sind und Clavulansäure kombiniert werden. Eine solche Kombination wäre z.B. die mit Oxacillin oder Dicloxacillin.

Die erfindungsgemäßen Verbindungen können zum Zweck der Erweiterung des Wirkungsspektrums und um eine Wirkungssteigerung zu erreichen auch mit Aminoglykosidantibiotica, wie z.B. Gentamicin, Sisomicin, Kanamicin, Amikacin oder Tobramicin kombiniert werden.

## Herstellungbeispiele

### Beispiel 1

(6S, 7S)-(D)-[(2-Aminobenzothiazol-6-yl)glycyl-amido]-3-chlor-1-azabicyclo-[4,2,0]oct-2-en-8-on-2-carbonsäure

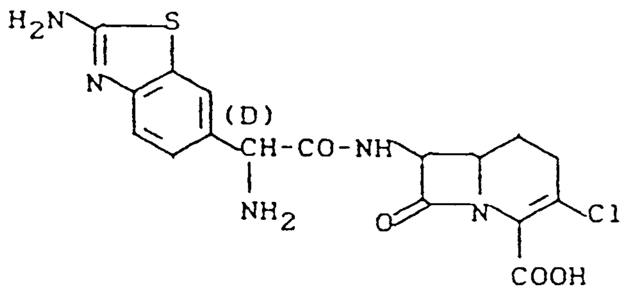

a) Aktivierung der Precursorsäure:

547 mg [entspricht 520 mg (1,44 mmol) Reinmaterial; 1,3 equivalente] Natrium-D-α-[(1-methyl-2-methoxycarbonylvinyl)-amino]-(2-aminobenzothiazol-6-yl)acetat werden in 4 ml Dimethylformamid und 1,5 ml Acetonitril klar gelöst. Dann kühlt man die Lösung auf -60 ° C und gibt nacheinander 1 Tropfen 3-Dimethylamino-1-propanol und 0,138 ml Chlorameisensäureethylester hinzu und rührt 30 min bei -60 ° C.

b) Zubereitung der Carbacephemkomponente:

0,300 mg (1,10 mmol) (±)-cis-7-Amino-3-chlor-1-azabicyclo-[4,2,0]oct-2-en-8-on-2-carbonsäure-t-butylester (siehe EP Patent 0 014476, Kyowa Hakko Kogyo Co. Ltd.] werden in 4 ml Dimethylformamid und 0,8 ml Acetonitril gelöst und 0,3 ml Wasser bei 0 ° C hinzugefügt.

c) Kupplung:

Die auf 0 ° C gekühlte Carbacephem-Lösung (b) gibt man langsam zur Lösung des gemischten Anhydrids (a) bei -60 ° C hinzu, wobei die Temperatur von -60 ° C auf -30 ° C ansteigt. Man rührt insgesamt 30 min nach und läßt die Temperatur der Reaktionslösung auf 0 ° C kommen. Anschließend gibt man 300 µl konzentrierte Salzsäure hinzu und rührt die Lösung 10 min bei 0 ° C.

d) Isolierung:

Man destilliert das organische Lösemittel ab und stellt mit 10%, Ammoniak-Lösung unter Eiskühlung auf pH 7,5. Man versetzt mit 20 ml Essigester und 10 ml 10%iger Natriumhydrogencarbonat-Lösung und rührt 5 min. Die Essigesterphase wird abgetrennt, einmal mit 5 ml gesättigter $NaHCO_3$-Lösung und zweimal mit 10 ml Wasser gewaschen. Danach wird die Essigesterphase über Natriumsulfat getrocknet, vom Trockenmittel abfiltriert und an Kieselgel (40 g, 0,04-0,063 mm) mit dem Elutionsmittel Essigester/n-Hexan = 1:5 chromatographiert. Der nach Vereinigen der Fraktionen und Abziehen des Solvens zurückbleibende Hartschaum wird in 4 ml Dichlormethan gelöst, auf 0 ° C gekühlt und mit einer Mischung von 4 ml Trifluoressigsäure und 0,3 ml Anisol versetzt. Die Lösung wird danach 20 min bei 0 ° C bis Raumtemperatur gerührt, anschließend zu einem Öl eingeengt, in Wasser gelöst und mit Ether gewaschen. Die wäßrige Phase wird auf eine RP-Säule (Hibar 250-25, Merck) gepumpt. Die Säule wird zunächst mit Wasser, danach mit 1 % bis 10 %igem Methanol eluiert.Die Fraktionen werden mittels analytischer HPLC untersucht und die Fraktionen, die das (6R, 7S)-Diastereomere enthalten, vereinigt, Methanol im Vakuum abdestilliert und die wäßrige Lösung lyophilisiert. Man erhält 52 mg als farbloses Lyophilisat.

$C_{17}H_{16}ClN_5O_4S$ x $2H_2O$ (457,89)

| | |
|---|---|
| NMR (DCOOD): δ = | 1,5 - 1,67 (m, 1H); 1,86 - 1,96 (m, 1H); 2,6 - 2,8 (m, 2H); 4,05 - 4,15 (mm, 1H); 5,55 (d, 1H, J = 419 Hz); 5,68 (s, 1H); 7,79 - 7,86 (q, 2H); 8,13 (s, 1H) ppm. |
| HPLC-analytisch: | Hibar 250-4, RP-8, 10 µm, 254 nm |
| Laufmittel : | Acetonitril/Eisessig/Wasser (100/30/870) |
| Fluß: | 3 ml $min^{-1}$ / Konz.: 1,0 ‰ |

Beispiel 2

( + )-cis-(D)-7-[(2-Aminobenzothiazol-6-yl)-glycyl-amido]-1-azabicyclo[4,2,0]-oct-2-en-8-on-2-carbonsäure

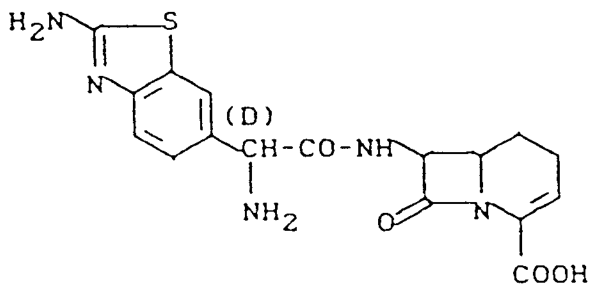

a) Aktivierung der Precursorsäure:

547 mg [entspricht 567 mg (1,57 mmol) Reinmaterial; 1,3 equivalente] Natrium-D-α-[(1-methyl-2-methoxycarbonylvinyl)-amino]-(2-aminobenzothiazol-6-yl)acetat (Gehalt = 95%) werden analog Beispiel 1a mit 0,151 ml (1,57 mmol) Chlorameisensäureethylester 30 min bei -60 °C aktiviert.

b) Zubereitung der Carbacephemkomponente:

0,220 mg (1,21 mmol) (±)-cis-7-Amino-1-azabicyclo[4,2,0]oct-2-en-8-on-2-carbonsäure [EP 0 014 476, Kyowa Hakko Kogyo Co. 1980] werden in 4 ml Dimethylformamid und 0,8 ml Acetonitril suspendiert und durch Zugabe von wenigen Tropfen 1 N Natronlauge bei Eiskühlung bis pH 8,1 in eine klare Lösung verwandelt.

c) Kupplung, Deblockierung und Isolierung des Betains

Die gekühlte Carbacephem-Lösung (0 °C) nach b) läßt man zur Lösung des gemischten Anhydrids der Precursorsäure nach a) bei -70 °C zutropfen und rührt 10 min bei -70 °C nach. Anschließend läßt man die Temperatur der Lösung innerhalb von 45 min auf 0 °C kommen und rührt mit 100 mg Kieselgur noch weitere 10 min. Danach saugt man die Reaktionsmischung über ein Seitzfilter, wäscht mit Dimethylformamid nach und versetzt das Filtrat mit 300 µl 12 N Salzsäure. Das Volumen der Lösung wird starkt konzentriert und das Filtrat mit 10%iger Ammoniak-Lösung auf pH 4,0 gestellt. Das Filtrat wird auf eine RP-18-Säule (Hibar 250-25, Merck) gepumpt. Die Säule wird zunächst mit Wasser und danach mit Wasser, zu dem man Aceton von 2% bis 10% hinzufügt, eluiert. Man sammelt 30 Fraktionen mit einem Volumen von jeweils 10 ml. Die Fraktionen, die das gewünschte Diastereomer in reiner Form enthalten, werden im Vakuum von Aceton abdestilliert und lyophilisiert.

Beispiel 3

(+)-cis-(D)-7-[(2-Aminobenzothiazol-6-yl)-glycyl-amido]-4-α-hydroxy-1-azabicyclo(4,2,0]oct-2-en-8-on-2-carbonsäure

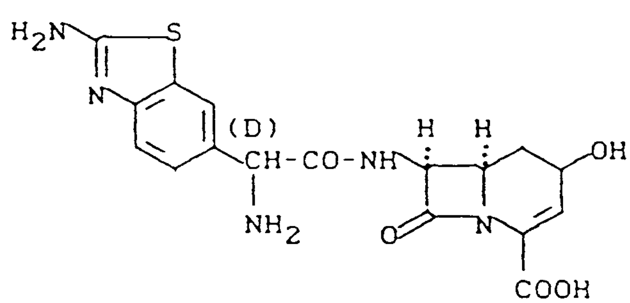

58,3 mg [entspricht 55,4 mg (0,153 mmol) Reinmaterial; 1,3 equivalente] Natrium-D-α-[1-methyl-2-methoxycarbonylvinyl)-amino]-(2-aminobenzothiazol-6-yl)-acetat (Gehalt = 95%) werden analog Beispiel 1a mit 14,7 µl Chlorameisensäureethylester in Dimethylformamid und Acetonitril in Gegenwart von katalytischen Mengen von 3-Dimethylamino-1-propanol mit 30 mg (0,117 mmol) (± )-cis-7β-Amino-4-α-hydroxy-1-azabicyclo[4,2,0]oct-2-en-8-on-2-carbonsäure-t-butylester (EP-Patent 0 025 602, Kyowa Hakko Kogyo Co. 1980) umgesetzt.

Der t-Butylester des Carbacephemderivates wird analog Beispiel 1d mit

Trifluoressigsäure/Methylenchlorid (1:1) behandelt. Das isolierte Trifluoracetat wird in Wasser gelöst und an Adsorber-Harz LPG 4429 (Lewatit® OC 1062) zunächst mit Wasser und anschließend mit wäßrigem Aceton mit einem steigenden Gehalt von 1% bis 10% Aceton chromatographiert.

Beispiel 4

(6R, 7S)-(D)-7-[(2-Amino-1H-benzimidazol-5-yl)glycylamido]-3-chlor-1-azabicyclo[4,2,0]oct-2-en-8-on-2-carbonsäure

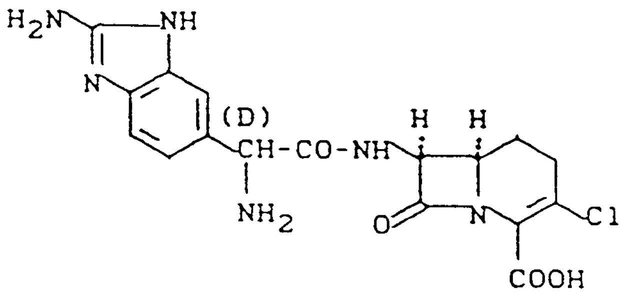

a) Kupplungsreaktion:

Eine Mischung von 1,0 g (2,61 mmol) (±)-cis-7-Amino-3-chlor-1-azabicyclo[4,2,0]oct-2-en-8-on-2-carbonsäurebenzhydrylester, 1,27 g (3,13 mmol, 1,2 equiv.) DL-α-t-Butyloxycarbonylamino-α-(2-t-butyloxycarbonyl-1H-benzimidazol-5-yl)-essigsäure und 0,646 g (3,13 mmol, 1,2 equiv.)-Dicyclohexylcarbodiimid (DCC) wird in 30 ml Tetrahydrofuran 2,5 h bei Raumtemperatur gerührt. Man saugt vom ausgefallenen Dicyclohexylharnstoff ab, wäscht mit Tetrahydrofuran nach und engt die Lösung bis zur Trockene ein. Der Rückstand wird in Essigester aufgenommen und nacheinander zweimal mit gesättigter Natriumhydrogencarbonat-Lösung, einmal mit Kochsalzlösung, einmal mit 10%iger Salzsäure und zum Schluß nochmals mit Kochsalzlösung gewaschen. Die Essigesterphase wird über Natriumsulfat getrocknet, filtriert und Essigester im Vakuum bis zur Trockene eingeengt.

b) Deblockierung und Diastereomerentrennung

Der Hartschaum wird in 5 ml Methylenchlorid gelöst, auf 0 °C gekühlt und nacheinander 0,5 ml Anisol und 5 ml Trifluoressigsäure hinzugegeben. Man rührt anschließend im Kältebad 45 min, engt die Lösung bis zur Trockene ein und versetzt den öligen Rückstand mit Ether, wobei das Trifluoracetatsalz auskristallisiert. Das Trifluoracetatsalz wird im Vakuum getrocknet, anschließend in 25 ml Wasser gelöst und über ein Membranfilter (Millipore, 45 µm) filtriert. Das Filtrat wird auf eine RP-18-Säule (Hibar 250-25, Merck) gepumpt. Die Säule wird zunächst mit 200 ml Wasser, danach mit Wasser, das kontinuierlich mit einem steigenden Gehalt von Methanol von 1 bis 15% versetzt wird, fortlaufend eluiert. Die Fraktionen werden mittels analytischer HPLC untersucht und die Eluate, die das gewünschte (6R, 7S)-(D)-Diastereomere enthalten, werden vereinigt. Methanol wird im Vakuum abdestilliert und die wäßrige Lösung lyophilisiert.

Beispiel 5

(6R, 7S)-(D)-7-[(2-Aminobenzoxazol-5-yl)glycyl-amido]-3-chlor-1-azabicyclo[4,2,0]oct-2-en-8-on-2-carbonsäure

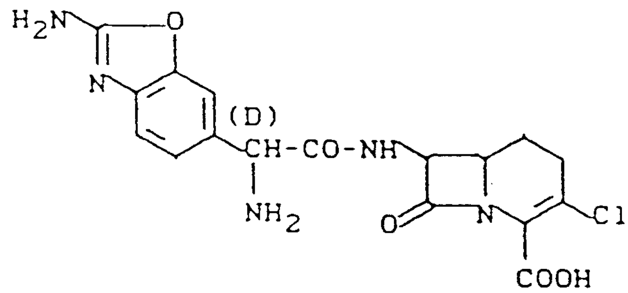

Eine Mischung von 0,8 g (2,1 mmol) (±)-cis-7-Amino-3-chlor-1-azabicyclo[4,2,0]-oct-2-en-8-on-2-carbonsäurebenzhydrylester, 0,82 g (2,52 mmol, 1,2 equiv.) D-α-Butyloxycarbonylamino-α-(2-amino-benzoxazol-5-yl)-essigsäure-monohydrat und 0,52 g (2,52 mmol, 1,2 equiv) DCC werden analog Beispiel 4 umgesetzt. Der Boc-geschützte Carbacephembenzhydrylester wird mit Trifluoressigsäure in Methylenchlorid analog Beispiel 4 deblockiert und das Trifluoracetat an einer RP-18-Säule chromatographiert.

Beispiel 6

(6R, 7S)-(D)-7-[(Benzofuryl-5-yl)glycyl-amido]-3-chlor-1-azabicyclo[4,2,0]oct-2-en-8-on-2-carbonsäure

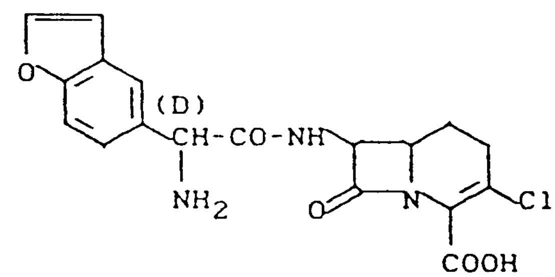

Eine Mischung von 0,650 g (1,7 mmol) (±)-cis-7-Amino-3-chlor-1-azabicyclo[4,2,0]oct-2-en-8-on-2-carbonsäurebenzhydrylester, 0,594 g (2,04 mmol, 1,2 equiv.) DL-α-(t-Butyloxycarbonylamino-α-benzofur-5-yl)-essigsäure und 0,421 g (2,04 mmol, 1,2 equiv.) DCC werden analog Beispiel 4 in Tetrahydrofuran umgesetzt. Der Boc-geschützte Carbacephembenzhydrylester wird mit Trifluoressigsäure in Methylenchlorid analog Beispiel 4 in das Trifluoracetat-Salz verwandelt. Das Trifluoracetat wird in Wasser unter Zusatz von Essigsäure gelöst, auf eine RP-18-Säule (Hibar 250-25, Merck) gepumpt und mit 3%iger Essigsäure, zu der man Methanol mit einem steigenden Anteil von 2% bis 20% hinzufügt, eluiert. Das Eluat, das die gewünschte Substanz enthält, wird nach Abdestillieren von Methanol gefriergetrocknet.

**Patentansprüche**

**1.** Heteroanellierte Phenylglycin-β-Lactam-Antibiotika der allgemeinen Formel (I)

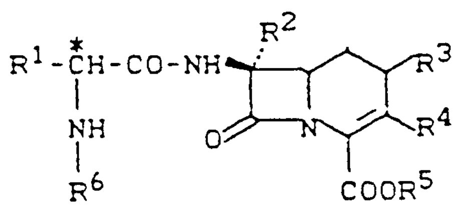

(I)

in welcher

$R^1$ - für eine Gruppe der Formel

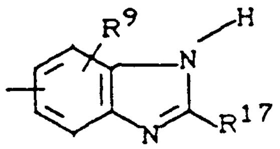 , 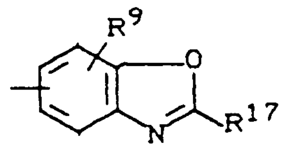 , 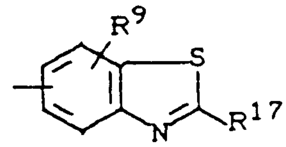

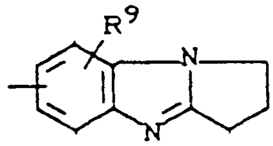 , 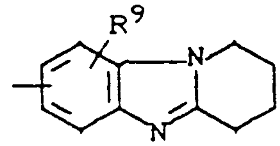 , 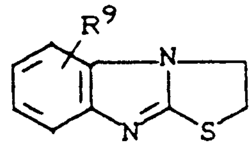

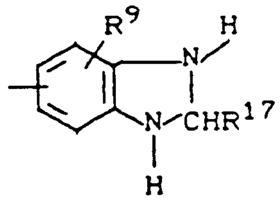 , 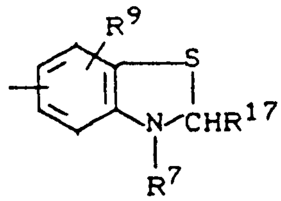 , 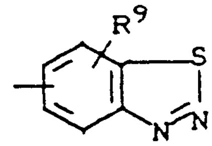 ,

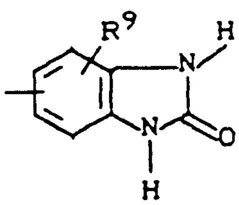 , 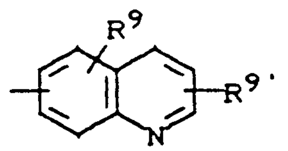 , 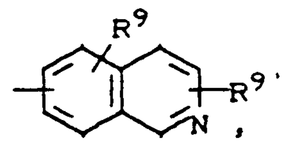 ,

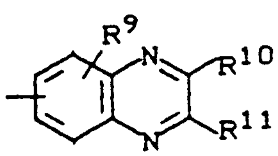 , 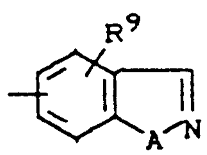 , 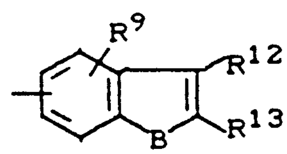

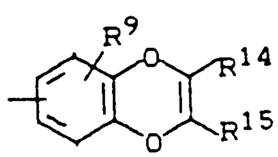

steht,
worin

$R^7$ - für Wasserstoff steht, oder
- für geradkettiges, verzweigtes oder cyclisches, gegebenenfalls durch Fluor, Amino, Hydroxy oder Phenyl substituiertes Alkyl oder Alkenyl mit bis zu 6 Kohlenstoffatomen steht,

$R^9$, $R^{9'}$ gleich oder verschieden sind und
- für Wasserstoff stehen, oder
- für geradkettiges oder verzweigtes Alkyl, Alkoxy oder Alkylthio mit jeweils bis zu 4 Kohlenstoffatomen stehen, oder
- für Trifluormethyl oder Trifluormethoxy stehen, oder
- für Hydroxy, Nitro, Cyano, Fluor oder Chlor stehen, oder
- für Amino, Methylamino, Dimethylamino,

$R^{10}$, $R^{11}$ gleich oder verschieden sind und für Wasserstoff stehen, oder
- für Phenyl stehen, das gegebenenfalls substituiert ist durch Chlor, Fluor, Alkyl mit bis zu 4 Kohlenstoffatomen, Methoxy, Methylthio, Trifluormethyl, Trifluormethoxy, Trifluormethylthio, Nitro, Cyano, Amino oder Dimethylamino, oder
- für Amino, Methylamino, Dimethylamino,

oder
- für Hydroxy stehen, oder
- für Alkoxy mit bis zu 4 Kohlenstoffatomen stehen, oder
- für Benzoyloxy oder Acetyloxy stehen, oder
- für geradkettiges, verzweigtes oder cyclisches Alkyl oder Alkenyl mit jeweils bis zu 6 Kohlenstoffatomen stehen, das substituiert sein kann, durch Fluor, Chlor, Methoxy, Methylthio, Trifluormethoxy oder Cyano,

$R^{12}$, $R^{13}$ gleich oder verschieden sind und
- für Wasserstoff stehen, oder
- für Phenyl stehen, das gegebenenfalls substituiert ist durch Fluor, Chlor, Alkyl mit bis zu 4 Kohlenstoffatomen, Methoxy, Methylthio, Trifluormethyl, Trifluormethoxy, Trifluormethylthio, Nitro, Cyano, Amino oder Dimethylamino, oder
- für Pyridyl, Thienyl, Furyl, Pyrimidyl oder Hydroxy stehen, oder
- für Amino, Methylamino, Dimethylamino, oder
- für Alkoxy mit bis zu 4 Kohlenstoffatomen stehen, oder
- für Benzoyloxy oder Acetyloxy stehen, oder
- für Benzoyl oder Acetyl stehen, oder
- für Alkoxycarbonyl mit bis zu 4 Kohlenstoffatomen stehen, oder
- für geradkettiges, verzweigtes oder cyclisches Alkyl oder Alkenyl mit bis zu 6 Kohlenstoffatomen stehen, das gegebenenfalls substituiert ist durch Fluor, Chlor, Methoxy, Methylthio, Trifluormethoxy, Cyano, Phenyloxy oder Benzyloxy,

$R^{14}$, $R^{15}$ gleich oder verschieden sind und
- für Wasserstoff stehen, oder
- für geradkettiges, verzweigtes oder cyclisches Alkyl oder Alkenyl mit bis zu 6 Kohlenstoffatomen stehen, das gegebenenfalls substituiert ist durch Fluor, Chlor, Methoxy, Methylthio, Trifluormethoxy oder Cyano, oder
- für Phenyl stehen, das gegebenenfalls substituiert ist durch Fluor, Chlor, Alkyl mit bis zu 4 Kohlenstoffatomen, Methoxy, Methylthio, Trifluormethyl, Trifluormethoxy, Trifluormethylthio, Nitro, Cyano, Amino oder Dimethylamino, oder
- für Alkoxycarbonyl mit bis zu 4 Kohlenstoffatomen stehen,

A - für O, S oder $-NR^{19}$ steht,

B - für O oder $-NR^{16}$ steht,

$R^{16}$ für Wasserstoff steht, oder
- für Phenyl steht, oder
- für geradkettiges oder verzweigtes Alkyl mit bis zu 4 Kohlenstoffatomen steht,

$R^{17}$ für Wasserstoff steht, oder
- für geradkettiges oder verzweigtes Alkyl oder Alkenyl mit jeweils bis zu 4 Kohlenstoffatomen steht, das gegebenenfalls substituiert ist durch Fluor, Chlor, Alkoxy mit bis zu 4 Kohlenstoffatomen, Hydroxy, Carboxy, Phenyl, Sulfo, Amino, Alkylamino, Dialkylamino mit jeweils bis zu 3 Kohlenstoffatomen je Alkylgruppe, Phenylamino, Benzylamino oder durch Acetylamino, oder
- für Fluor, Chlor oder Brom steht, oder
- für Alkoxy oder Alkylthio mit jeweils bis zu 4 Kohlenstoffatomen steht, oder
- für Phenyl steht, oder
- für Amino, Alkylamino, Dialkylamino mit jeweils bis zu 3 Kohlenstoffatomen je Alkylgruppe,

$R^{18}$ - die gleiche Bedeutung hat wie $R^{17}$ und mit diesem gleich oder verschieden sein kann und

$R^{19}$ - die gleiche Bedeutung hat wie $R^{16}$ und mit diesem gleich oder verschieden ist,

$R^2$ - für Wasserstoff steht, oder
- für Methoxy steht, oder
- für Formamido oder Hydroxylamino steht,

$R^3$ - für Wasserstoff, Hydroxy, Methyl oder Methoxy steht,

$R^4$ - für Wasserstoff, Chlor, Methyl, Methoxy, Methoxymethyl, Vinyl, cis-Propenyl oder Acetyloxymethyl steht, oder

$R^5$ - für Wasserstoff steht, oder
- für einen Rest der Formel $-CH_2-OCOCH_3$,

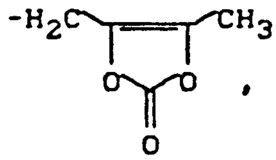

$-CH(CH_3)-OCOOC_2H_5$ oder $-CH_2-OCO-C(CH_3)_3$ steht,
und

$R^6$ - für Wasserstoff steht,
und deren pharmazeutisch verträgliche Natriumsalze.

**2.** Verfahren zur Herstellung der heteroanellierten Phenylglycin-β-Lactam-Antibiotika der allgemeinen Formel (I) nach Anspruch 1, dadurch gekennzeichnet, daß man Verbindungen der Formel (II)

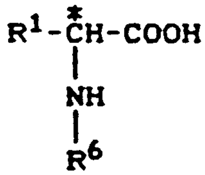

(II)

in welcher
$R^1$ und $R^6$ die im Anspruch 1 angegebene Bedeutung haben,
nach Aktivierung der Carboxylgruppe durch Überführung in ein gemischtes Anhydrid, beispielsweise mit Chlorameisensäureethylester oder Chlorameisensäureisobutylester oder Methansulfonsäurechlorid, oder durch Überführen in das Säurehalogenid, oder durch Überführen in einen aktivierten Ester, beispielsweise mit Dicyclohexylcarbodiimid (DCC), gegebenenfalls in Anwesenheit von N-Hydroxybenztriazol,
mit den β-Lactamaminen der allgemeinen Formel (III),

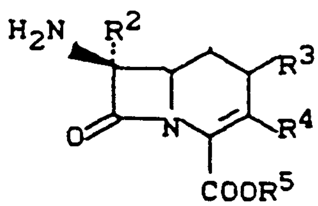

(III)

in welcher
$R^2$, $R^3$, $R^4$ und $R^5$ die im Anspruch 1 angegebene Bedeutung haben,
umsetzt,
dann gegebenenfalls Schutzgruppen abspaltet und die gewünschten Salze oder aus den Salzen die freien Säuren herstellt.

**3.** Heteroanellierte Phenylglycin-β-Lactam-Antibiotika der allgemeinen Formel (I) nach Anspruch 1 zur Bekämpfung von Krankheiten.

**4.** Arzneimittel enthaltend heteroanellierte Phenylglycin-β-Lactam-Antibiotika der allgemeinen Formel (I) nach Anspruch 1.

**5.** Verwendung von heteroanellierten Phenylglycin-β-Lactam-Antibiotika der allgemeinen Formel (I) nach Anspruch 1 zur Herstellung von Arzneimitteln.

**Claims**

**1.** Heteroanellated phenylglycine-$\beta$-lactam antibiotics of the general formula (I)

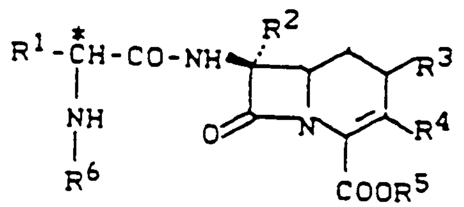

(I)

in which

$R^1$ - represents a group of formula

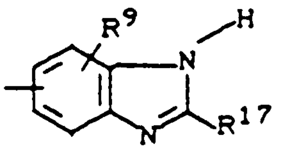 , 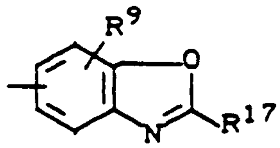 , 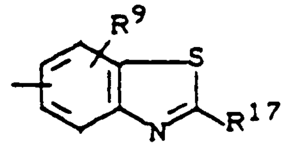

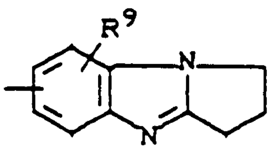 , 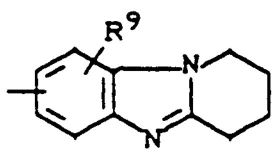 , 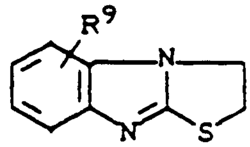

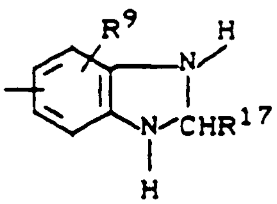 , 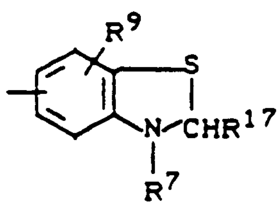 , 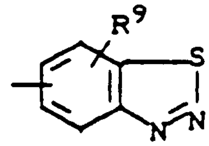 ,

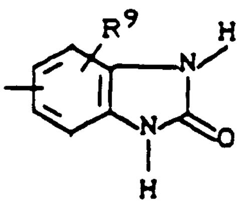 , 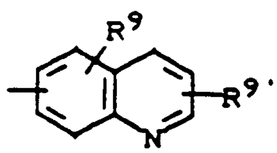 , 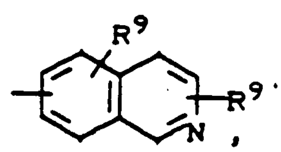 ,

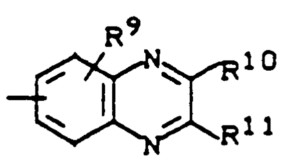 , 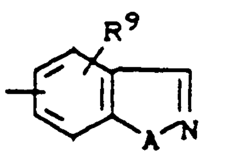 , 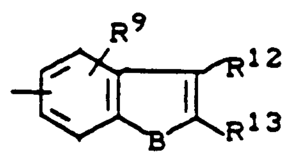

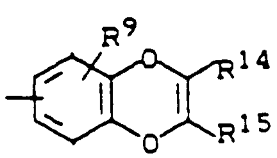

wherein

$R^7$ - represents hydrogen, or

- represents straight-chain, branched or cyclic alkyl or alkenyl having up to 6 carbon atoms, each of which is optionally substituted by fluorine, amino, hydroxyl or phenyl,

$R^9$ and $R^{9'}$ are identical or different and
- represent hydrogen, or
- represent straight-chain or branched alkyl, alkoxy or alkylthio each having up to 4 carbon atoms, or
- represent trifluoromethyl or trifluoromethoxy, or
- represent hydroxyl, nitro, cyano, fluorine or chlorine, or
- represent amino, methylamino, dimethylamino,

$R^{10}$ and $R^{11}$ are identical or different and represent hydrogen, or
- represent phenyl which is optionally substituted by chlorine, fluorine, alkyl having up to 4 carbon atoms, methoxy, methylthio, trifluoromethyl, trifluoromethoxy, trifluoromethylthio, nitro, cyano, amino or dimethylamino, or
- represent amino, methylamino, dimethylamino, or
- represent hydroxyl, or
- represent alkoxy having up to 4 carbon atoms, or
- represent benzoyloxy or acetyloxy, or
- represent straight-chain, branched or cyclic alkyl or alkenyl each having up to 6 carbon atoms, each of which can be substituted by fluorine, chlorine, methoxy, methylthio, trifluoromethoxy or cyano,

$R^{12}$ and $R^{13}$ are identical or different and
- represent hydrogen, or
- represent phenyl which is optionally substituted by fluorine, chlorine, alkyl having up to 4 carbon atoms, methoxy, methylthio, trifluoromethyl, trifluoromethoxy, trifluoromethylthio, nitro, cyano, amino or dimethylamino, or
- represent pyridyl, thienyl, furyl, pyrimidyl or hydroxyl, or
- represent amino, methylamino, dimethylamino, or
- represent alkoxy having up to 4 carbon atoms, or
- represent benzoyloxy or acetyloxy, or
- represent benzoyl or acetyl, or
- represent alkoxycarbonyl having up to 4 carbon atoms, or
- represent straight-chain, branched or cyclic alkyl or alkenyl having up to 6 carbon atoms, each of which is optionally substituted by fluorine, chorine, methoxy, methylthio, trifluoromethoxy, cyano, phenyloxy or benzyloxy,

$R^{14}$ and $R^{15}$ are identical or different and
- represent hydrogen, or
- represent straight-chain, branched or cyclic alkyl or alkenyl having up to 6 carbon atoms, each of which is optionally substituted by fluorine, chlorine, methoxy, methylthio, trifluoromethoxy or cyano, or
- represent phenyl which is optionally substituted by fluorine, chlorine, alkyl having up to 4 carbon atoms, methoxy, methylthio, trifluoromethyl, trifluoromethoxy, trifluoromethylthio, nitro, cyano, amino or dimethylamino, or
- represent alkoxycarbonyl having up to 4 carbon atoms,

A - represents O, S or $-NR^{19}$,

B - represents O or $-NR^{16}$,

$R^{16}$ represents hydrogen, or
- represents phenyl, or
- represents straight-chain or branched alkyl having up to 4 carbon atoms,

$R^{17}$ represents hydrogen, or
- represents straight-chain or branched alkyl or alkenyl each having up to 4 carbon atoms, each of which is optionally substituted by fluorine, chlorine, alkoxy having up to 4 carbon atoms, hydroxyl, carboxyl, phenyl, sulpho, amino, alkylamino or dialkylamino each having up to 3 carbon atoms per alkyl group, phenylamino, benzylamino or by acetylamino, or
- represents fluorine, chlorine or bromine, or
- represents alkoxy or alkylthio each having up to 4 carbon atoms, or
- represents phenyl, or

- represents amino, alkylamino or dialkylamino each having up to 3 carbon atoms per alkyl group,

$R^{18}$ - has the same meaning as $R^{17}$ and can be identical to or different from this and

$R^{19}$ - has the same meaning as $R^{16}$ and is identical to or different from this,

$R^2$ - represents hydrogen, or
- represents methoxy, or
- represents formamido or hydroxylamino,

$R^3$ - represents hydrogen, hydroxyl, methyl or methoxy,

$R^4$ - represents hydrogen, chlorine, methyl, methoxy, methoxymethyl, vinyl, cis-propenyl or acetyloxymethyl, or

$R^5$ - represents hydrogen, or
- represents a radical of the formula $-CH_2-OCOCH_3$,

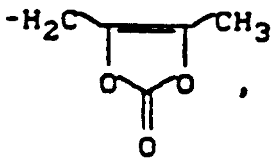

$-CH(CH_3)-OCOOC_2H_5$ or $-CH_2-OCO-C(CH_3)_3$
and

$R^6$ - represents hydrogen,

and their pharmaceutically tolerable sodium salts.

**2.** Process for the preparation of the heteroanellated phenylglycine-$\beta$-lactam antibiotics of the general formula (I) according to Claim 1, characterised in that compounds of the formula (II)

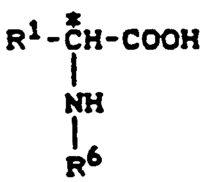

(II)

in which
$R^1$ and $R^6$ have the meaning given in Claim 1,
are reacted, after activation of the carboxyl group by conversion into a mixed anhydride, for example using ethyl chloroformate or isobutyl chloroformate or methanesulphonyl chloride, or by conversion into the acid halide, or by conversion into an activated ester, for example using dicyclohexylcarbodiimide (DCC), if appropriate in the presence of N-hydroxybenzotriazole,
with the $\beta$-lactam amines of the general formula (III)

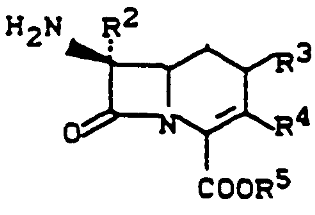

(III)

in which
$R^2$, $R^3$, $R^4$ and $R^5$ have the meaning given in Claim 1,
and then, if desired, eliminating protecting groups and preparing the desired salts or the free acids from the salts.

3. Heteroanellated phenylglycine-β-lactam antibiotics of the general formula (I) according to Claim 1 for combating diseases.

4. Medicaments containing heteroanellated phenylglycine-β-lactam antibiotics of the general formula (I) according to Claim 1.

5. Use of heteroanellated phenylglycine-β-lactam antibiotics of the general formula (I) according to Claim 1 for the preparation of medicaments.

## Revendications

1. Antibiotiques hétérocondensés du type phénylglycine-β-lactame de formule générale (I)

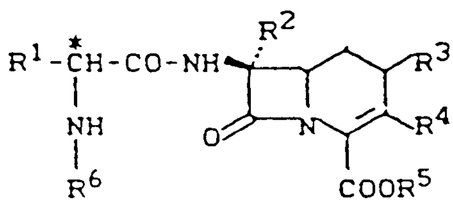

(I)

dans laquelle

$R^1$ représente un groupe de formule

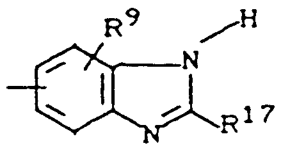 , 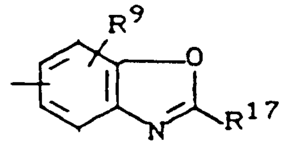 , 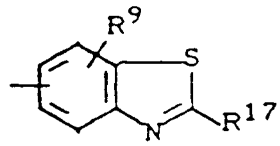

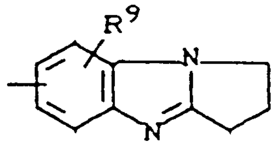 , 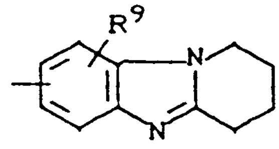 , 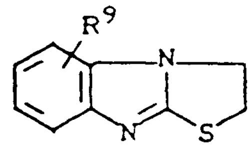

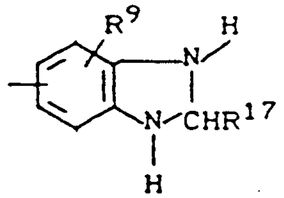 , 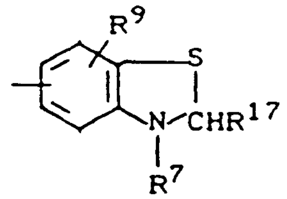 , 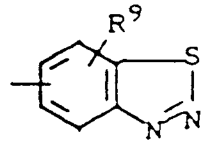 ,

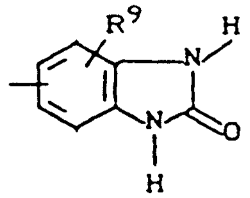 , 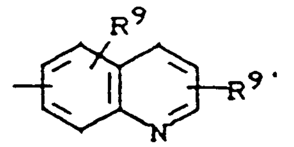 , 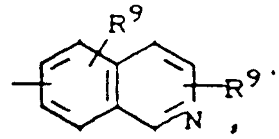 ,

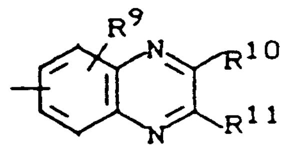 , 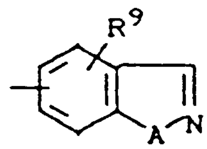 , 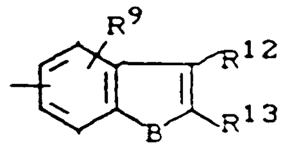

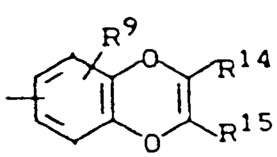

où

$R^7$ représente l'hydrogène, ou bien
un groupe alkyle ou alcényle ayant jusqu'à 6 atomes de carbone, à chaîne droite, à chaîne ramifiée ou cyclique, éventuellement substitué par du fluor, un radical amino, hydroxy ou phényle,

$R^9$, $R^{9'}$ sont identiques ou différents et représentent
- l'hydrogène, ou
- un groupe alkyle, alkoxy ou alkylthio à chaîne droite ou ramifiée ayant chacun jusqu'à 4 atomes de carbone, ou
- un groupe trifluorométhyle ou trifluorométhoxy, ou
- un groupe hydroxy, nitro, cyano, du fluor ou du chlore, ou
- un groupe amino, méthylamino, diméthylamino,

$R^{10}$, $R^{11}$ sont identiques ou différents et représentent l'hydrogène ou
- un groupe phényle qui est éventuellement substitué par du chlore, du fluor, un radical alkyle ayant jusqu'à 4 atomes de carbone, méthoxy, méthylthio, trifluorométhyle, trifluorométhoxy, trifluorométhylthio, nitro, cyano, amino ou diméthylamino, ou
- un groupe amino, méthylamino, diméthylamino, ou
- un groupe hydroxy, ou
- un groupe alkoxy ayant jusqu'à 4 atomes de carbone, ou

- un groupe benzoyloxy ou acétyloxy,
ou
- un groupe alkyle ou alcényle ayant jusqu'à 6 atomes de carbone à chaîne droite, à chaîne ramifiée ou cyclique, qui peut être substitué par du fluor, du chlore, un radical méthoxy, méthylthio, trifluorométhoxy ou cyano,

$R^{12}$, $R^{13}$ sont identiques ou différents et représentent
- l'hydrogène ou
- un groupe phényle qui est éventuellement substitué par du fluor, du chlore, un radical alkyle ayant jusqu'à 4 atomes de carbone, méthoxy, méthylthio, trifluorométhyle, trifluorométhoxy, trifluorométhylthio, nitro, cyano, amino ou diméthylamino, ou
- un groupe pyridyle, thiényle, furyle, pyrimidyle ou hydroxy, ou
- un groupe amino, méthylamino, diméthylamino ou
- un groupe alkoxy ayant jusqu'à 4 atomes de carbone, ou
- un groupe benzoyloxy ou acétyloxy, ou
- un groupe benzoyle ou acétyle, ou
- un groupe alkoxycarbonyle ayant jusqu'à 4 atomes de carbone, ou
- un groupe alkyle ou alcényle ayant jusqu'à 6 atomes de carbone, à chaîne droite, à chaîne ramifiée ou cyclique, qui est éventuellement substitué par du fluor, du chlore, un radical méthoxy, méthylthio, trifluorométhoxy, cyano, phényloxy ou benzyloxy,

$R^{14}$, $R^{15}$ sont identiques ou différents et représentent
- l'hydrogène ou
- un groupe alkyle ou alcényle ayant jusqu'à 6 atomes de carbone, à chaîne droite ou à chaîne ramifiée ou cyclique, qui est éventuellement substitué par du fluor, du chlore, un radical méthoxy, méthylthio, trifluorométhoxy ou cyano, ou
- un groupe phényle qui est substitué le cas échéant par du fluor, du chlore, un radical alkyle ayant jusqu'à 4 atomes de carbone, méthoxy, méthylthio, trifluorométhyle, trifluorométhoxy, trifluorométhylthio, nitro, cyano, amino ou diméthylamino ou
- un groupe alkoxycarbonyle ayant jusqu'à 4 atomes de carbone,

A représente O, S ou un groupe $-NR^{19}$,

B représente O ou un groupe $-NR^{16}$,

$R^{16}$ représente l'hydrogène ou
- un groupe phényle, ou
- un groupe alkyle à chaîne droite ou ramifiée ayant jusqu'à 4 atomes de carbone

$R^{17}$ représente l'hydrogène ou
- un groupe alkyle ou alcényle ayant chacun jusqu'à 4 atomes de carbone, à chaîne droite ou ramifiée, qui est substitué le cas échéant par du fluor, du chlore, un radical alkoxy ayant jusqu'à 4 atomes de carbone, hydroxy, carboxy, phényle, sulfo, amino, alkylamino, dialkylamino ayant chacun jusqu'à 3 atomes de carbone par groupe alkyle, par un radical phénylamino, benzylamino ou acétylamino ou
- du fluor, du chlore ou du brome, ou
- un groupe alkoxy ou alkylthio ayant chacun jusqu'à 4 atomes de carbone, ou
- un groupe phényle, ou
- un groupe amino, alkylamino, dialkylamino ayant chacun jusqu'à 3 atomes de carbone par groupe alkyle,

$R^{18}$ a la même définition que $R^{17}$ et peut être identique à ce dernier ou peut en être différent et

$R^{19}$ a la même définition que $R^{16}$ et peut être identique à ce dernier ou peut en être différent,

$R^2$ représente l'hydrogène ou
- un groupe méthoxy, ou
- un groupe formamido ou hydroxylamino,

$R^3$ représente l'hydrogène, un groupe hydroxy, méthyle ou méthoxy,

$R^4$ est l'hydrogène, le chlore, un groupe méthyle, méthoxy, méthoxyméthyle, vinyle, cis-propényle ou acétyloxyméthyle, ou

$R^5$ représente l'hydrogène ou
- un reste de formule

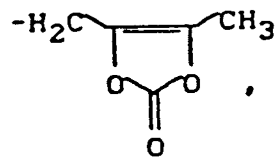,

$-CH(CH_3)-OCOOC_2H_5$ ou $-CH_2-OCO-C(CH_3)_3$,
et

$R^6$ représente l'hydrogène,
et leurs sels de sodium acceptables du point de vue pharmaceutique.

**2.** Procédé de production des antibiotiques hétérocondensés du type phénylglycine-$\beta$-lactame, de formule générale (I) suivant la revendication 1, caractérisé en ce qu'on fait réagir des composés de formule (II)

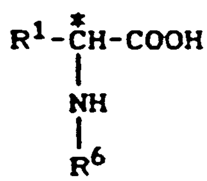

(II)

dans laquelle
$R^1$ et $R^6$ ont la définition indiquée dans la revendication 1,
après activation du groupe carboxyle par transformation en un anhydride mixte, par exemple avec l'ester éthylique de l'acide chloroformique ou l'ester isobutylique de l'acide chloroformique ou le chlorure d'acide méthanesulfonique, ou par transformation en l'halogénure d'acide ou par transformation en un ester activé, par exemple le dicyclohexylcarbodiimide (DCC), le cas échéant en présence de N-hydroxybenzotriazole,
avec les $\beta$-lactames-amines de formule générale (III),

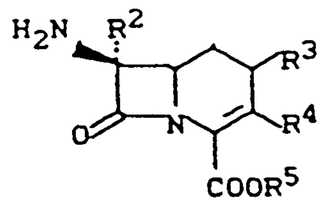

(III)

dans laquelle
$R^2$, $R^3$, $R^4$ et $R^5$ ont la définition indiquée dans la revendication 1,
puis on élimine éventuellement les groupes protecteurs et on prépare les sels désirés ou les acides libres à partir des sels.

**3.** Antibiotiques hétérocondensés du type phénylglycine-$\beta$-lactame de formule (I) suivant la revendication 1, destinés à combattre des maladies.

**4.** Médicaments contenant des antibiotiques hétérocondensés du type phénylgycine-$\beta$-lactame de formule générale (I) suivant la revendication 1.

**5.** Utilisation d'antibiotiques hétérocondensés du type phénylglycine-$\beta$-lactame de formule générale (I) suivant la revendication 1 pour la préparation de médicaments.